# EUROPÄISCHE PATENTANMELDUNG

(11) **EP 1 090 606 A2**
(43) Veröffentlichungstag der Anmeldung: **11.04.2001**
(21) Anmeldenummer: 00890255.3
(22) Anmeldetag: 22.08.2000
(51) Int. Cl.: A61C 8/00

(54) **Schutzschablone für ein Kieferimplantat**

(30) Priorität: 05.10.1999 AT 169799
(71) Anmelder: Watzek, Georg, Dr., 1080 Wien (AT)
(72) Erfinder: Watzek, Georg, Dr., 1080 Wien (AT)
(74) Vertreter: Kliment, Peter

(57) **Zusammenfassung**

Vorrichtung zum Ausschluß einer Verletzung von im Kieferknochen liegenden Implantaten oder Zahnwurzelanteilen. UM einen bzw. mehrere Schnitte im Kieferknochen anzubringen, ohne dass die Gefahr besteht, im Kieferknochen befindliche Objekte, wie beispielsweise Kieferimplantate oder Zähne samt Wurzeln zu beschädigen bzw. zu verletzen, ist vorgesehen, dass im oberen Endbereich eines Implantats (1) oder eines Zahns eine Platte (5) angeordnet ist, deren freier Endbereich gegen den Kieferknochen (2) gerichtet ist.

## Beschreibung

Die vorliegende Erfindung bezieht sich auf eine Vorrichtung zur Abdeckung und Sicherung eines Kieferimplantats bzw. eines Zahns während eines Kieferschneidevorganges gemäß Oberbegriff des Anspruchs 1.

Bei Operationen im Kieferbereich ist es sehr oft erforderlich, den Kieferknochen aufzuschneiden bzw. einzuschneiden. Wird beispielsweise ein ausgefallener Zahn im menschlichen Kiefer nicht ersetzt, bildet sich in der entstandenen Zahnlücke der Kieferknochen zurück, wodurch ein sogenannter Substanzdefekt im Kiefer entsteht. Soll bei einem solchen, bereits vorhandenen Knochendefizit ein Zahnersatz geschaffen werden, so kann auf Wunsch des Patienten ein Kieferimplantat in das menschliche Kiefer (Ober- oder Unterkiefer) implantiert werden.

Neuere Operationsmethoden erfordern nach dem Implantieren dieses Kieferimplantats im Kieferknochen das Freischneiden des Kieferimplantats samt eines Teiles des das Kieferimplantat umgebenden Kieferknochens, so dass durch langsame, schrittweise Dehnung des Kieferknochens (Aufdehnung des Spaltes, der durch das Freischneiden des das Kieferimplantat umgebenden Kieferknochens entstanden ist) eine Knochensubstanznachbildung gefördert wird.

Aber auch andere Probleme erfordern das Anbringen von Schnitten im Kieferknochen wie beispielsweise Knochendurchschneidungen bei Fehlbiß oder bei Entnahme von Knochentransplantaten.

Problematisch bei solchen Schnittvorgängen im Kiefer ist die Tatsache, dass durch unsachgemäße bzw. falsche Schnittführung das Kieferimplantat bzw. der Zahn und dessen Wurzel verletzt bzw. beschädigt werden können. Dies hätte jedoch schwerwiegende Folgen für den Patienten.

Ziel der vorliegenden Erfindung ist daher eine Vorrichtung, die es ermöglicht, einen bzw. mehrere Schnitte im Kieferknochen anzubringen, ohne dass die Gefahr besteht, im Kieferknochen befindliche Objekte, wie beispielsweise Kieferimplantate oder Zähne samt Wurzeln zu beschädigen bzw. zu verletzen.

Erfindungsgemäß wird dies durch die kennzeichnenden Merkmale des Anspruchs 1 erreicht.

Dadurch ist es möglich die Platte, welche vorzugsweise eine Metallplatte ist, so zu befestigen, dass sie schützend vor jenem Objekt liegt, in dessen umgebenden Bereich in den Kieferknochen geschnitten wird. Die Abmessungen der Platte sind dabei auf die Abmessungen des Kieferimplantats bzw. des Zahns samt Wurzeln abgestimmt, die vor einer unbeabsichtigten Verletzung geschützt werden sollen.

Durch die Merkmale des Anspruchs 2 ist es möglich einfache Winkel als Schutzplatte zu verwenden, womit auch eine einfache Herstellung der erfindungsgemäßen Vorrichtung möglich ist.

Durch das Merkmal des Anspruchs 3 kann eine alternative Befestigung der Platte an einem Kieferimplantat vorgesehen werden. Da solche Implantate ein Innengewinde aufweisen, in welchem im endgültigen Zustand dann der eigentliche Zahnersatz eingeschraubt ist, kann das Innengewinde vorerst auch mit einer einfachen Schraube verschraubt werden, wobei diese durch die erfindungsgemäße Bohrung der Platte gesteckt ist. Auf diese Art und Weise kann eine einfache Befestigung der Platte am Implantat erreicht werden.

Das Merkmal des Anspruchs 4 beschreibt eine alternative Lösung der Ausbildung der Platte.

Durch das Merkmal des Anspruchs 5 kann die Platte entsprechend der Form des Kieferknochens optimal gebogen und eingestellt werden.

Im folgenden erfolgt nun eine detaillierte Beschreibung der erfindungsgemäßen Vorrichtung. Dabei zeigt:
- Fig. 1: eine Seitenansicht einer erfindungsgemäßen Platte an einem Implantat befestigt
- Fig. 2: eine Frontansicht einer erfindungsgemäßen Platte an einem Implantat befestigt
- Fig. 3: eine Draufsicht einer erfindungsgemäßen Platte an einem Implantat befestigt
- Fig. 4: eine alternative Ausführungsform einer erfindungsgemäßen Platte an einem Implantat befestigt

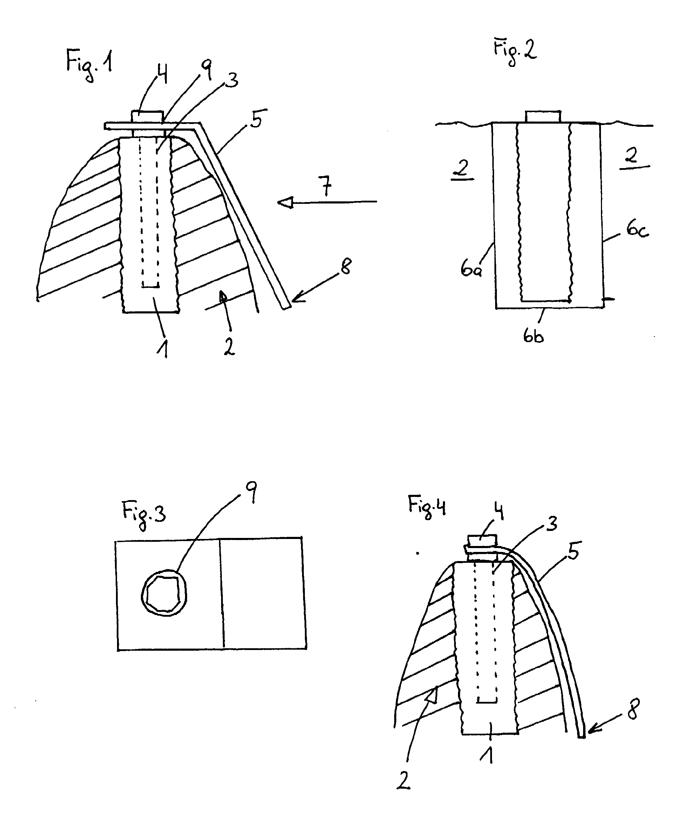

Im Ausführungsbeispiel in Fig. 1 ist ein ein Innengewinde 3 aufweisendes Kieferimplantat 1 fix in einen Kieferknochen 2 implantiert. In das Kieferimplantat 1 ist eine Schraube 4 geschraubt, an deren oberem Endbereich eine erfindungsgemäße Platte 5 befestigt ist. Die Platte 5 ist vorzugsweise aus Metall gefertigt und hat im vorliegenden Ausführungsbeispiel einen im wesentlichen L-förmigen Querschnitt. Es ist jedoch auch durchaus denkbar, anders geformte Platten 5, wie beispielsweise in Fig. 4 dargestellt, vorzusehen, wobei diese auch aus Kunststoff hergestellt sein können. Die Platte 5 ist dabei derart flexibel biegbar, dass ihre Querschnittsform der Form bzw. Dicke des Kieferknochens 2 angepasst werden kann.

Fig. 2 zeigt eine Ansicht der erfindungsgemäßen Platte 5 in Richtung des Pfeiles 7 aus Fig. 1. Die Abmessungen der Platte 5 sind so gewählt, dass das Kieferimplantat 1 bzw., alternativ dazu, der Zahn und dessen Wurzel vollkommen dahinter verborgen sind. Weiters kann durch größer Abmessungen der Platte 5 (wie in Fig. 2 ersichtlich) auch ein zusätzliches Stück des Kieferknochens, welcher das Kieferimplantat 1 bzw. den Zahn samt Wurzel umgibt, abgedeckt werden. Dies ist beispielsweise dann erforderlich, wenn gewährleistet werden soll, das ein Stück des Kieferknochens am Kieferimplantat bzw. am Zahn samt Wurzel haften bleiben soll.

Die Befestigung der erfindungsgemäßen Platte muß dabei nicht unbedingt an einer Schraube 4, welche in das Kieferimplantat 1 geschraubt ist, erfolgen, sondern kann auch direkt im oberen Endbereich des Kieferimplantats erfolgen. Aufgrund der Tatsache, dass das Kieferimplantat 1 jedoch meistens zur Gänze im Kieferknochen implantiert ist, bildet jene Variante, bei welcher die erfindungsgemäße Platte 5 an einer im das Implantat 1 geschraubten Schraube 4 befestigt ist, die bevorzugte Lösung.

Auch ist durchaus vorstellbar die erfindungsgemäße Platte 5 an einem oder mehreren Zähnen zu befestigen um den gewünschten Schutzeffekt zu erzielen.

Die freien Kanten 6a,6b,6c der erfindungsgemäßen Platte 5 können in Sonderfällen auch als Schnittführungskanten für das Schneidinstrument verwendet werden. Dadurch können exakte Schnitte im Kieferknochen getätigt werden. Die Ausbildung der freien Kanten 6a,6b,6c kann dabei durchaus auch eine andere Form als in Fig. 2 dargestellt haben. So ist prinzipiell jede Form möglich, wobei die Form entsprechend dem Zweck des Kieferschnittes ausgewählt wird.

## Patentansprüche

1. Vorrichtung zur Abdeckung und zum Schutz eines Kieferimplantats (1) bzw. eines Zahnes samt Wurzeln während eines Schneidevorganges im Kieferknochen, **dadurch gekennzeichnet, dass** die Vorrichtung aus einer geknickten oder gekrümmten Platte (5) besteht, deren einer Endbereich im oberen Endbereich eines Kieferimplantats (1) oder eines Zahns befestigbar ist und deren anderer freier Endbereich (8) gegen den Kieferknochen (2) gerichtet ist.

2. Vorrichtung nach Anspruch 1, **dadurch gekennzeichnet**, **dass** die Platte (5) im wesentlichen L-förmigen Querschnitt aufweist ist, wobei der kleinere Arm im wesentlichen rechtwinkelig zur Längsachse des Implantats (1) bzw. des Zahns anbringbar ist und der daran anschließende längere Arm unter einem Winkel zur Längsachse des Implantats (1) bzw. des Zahns angeordnet ist.

3. Vorrichtung nach Anspruch 1 und 2, **dadurch gekennzeichnet, dass** die Platte (5) in jenem Endbereich, der mit dem Implantat (1) bzw. dem Zahn verbunden ist, eine Bohrung (9) aufweist, durch welche eine Schraube (4), die mit dem Implantat (1) verschraubt ist, durchsteckbar ist.

4. Vorrichtung nach Anspruch 1, **dadurch gekennzeichnet, dass** die Platte (5) von jenem Bereich, in welchem sie mit dem Implantat (1) bzw. dem Zahn verbunden ist, kontinuierlich bis hin zum freien, gegen den Kieferknochen (2) gerichteten Endbereich (8), gebogen ist.

5. Vorrichtung nach einem der Ansprüche 1 bis 4, **dadurch gekennzeichnet, dass** die Platte (5) flexibel biegbar ist.
